# EUROPEAN PATENT SPECIFICATION

(11) **EP 2 900 308 B1**
(45) Date of publication and mention of the grant of the patent: **31.05.2017**
(21) Application number: 13808212.8
(22) Date of filing: 19.09.2013
(51) Int. Cl.: A61M 25/00, A61M 16/04

(54) **SELF POSITIONING TRACHEAL TUBE CLEARANCE MECHANISM USING WHISKS**
SELBSTPOSITIONIERENDER TRACHEALTUBUS-REINIGUNGSMECHANISMUS MIT WEDELN
MÉCANISME DE NETTOYAGE D'UN TUBE TRACHÉAL À AUTO-POSITIONNEMENT, POURVU DE CERCEAUX

(30) Priority: 28.09.2012 US 201261707259 P; 13.09.2013 US 201314026139
(43) Date of publication of application: 05.08.2015
(73) Proprietor: Avent, Inc., Alpharetta, GA 30004 (US)
(72) Inventor: STADELMAN, Jennifer, S., Alpharetta, Georgia 30009 (US); HERSHEY, Adrienne, A., Cumming, GA 30041 (US); CESA, Joseph, A., Cumming, GA 30040 (US); HEADLEY, JR., F., Anthony, Atlanta, GA 30342 (US); BREWER, John, Marietta, GA 30062 (US)
(74) Representative: Davies, Christopher Robert
(86) International application number: PCT/IB2013/058675
(87) International publication number: WO 2014/049497

(56) References cited:
- WO-A1-94/03226
- DE-A1-102010 026 774
- US-A1- 2007 038 226
- US-A1- 2011 023 885
- US-A1- 2011 186 052

## Description

The present disclosure relates to cleaning mechanisms for the central (breathing) lumen of tracheal tubes.

Tracheal intubation involves the insertion of a hollow tubular device, known as a tracheal tube, into the trachea of a patient. The tube may be inserted through the mouth or, less desirably, the nose or may be inserted through the neck by way of an incision in the front of the throat. If inserted through the mouth or nose the tube is referred to as an endotracheal tube, if through the front of the throat the tube is referred to as a tracheostomy or trach tube. The two types of tubes will be referred to as tracheal tubes herein. The tracheal tube passes into the trachea and terminates at a position above the carina, anterior to a position between the second and fourth thoracic vertebrate. Gases may then be introduced through the central lumen of the tracheal tube and into the lungs of the patient.

The primary purpose of tracheal intubation is to mechanically ventilate the patient's lungs when the patient is incapable of normal breathing induced ventilation. Intubation may also be used to apply anesthetic gases during surgical intervention. It is desirable to seal the passageway around the tracheal tube in order to maintain enough air pressure to force the air into the lungs during mechanical ventilation and to prevent escape of gases past the tube (i.e. "short circuiting" or bypassing of the lungs). Such a seal may be produced by the use of an inflatable cuff or balloon surrounding the tracheal tube near its distal end. When the tracheal tube has been introduced into the patient's trachea, the inflatable cuff will normally be located about 3 to 5 centimeters above the carina and within the tube-like trachea.

Once inflated, the cuff will engage the wall of the trachea and thereby seal the trachea and prevent the gases being introduced through the tracheal tube from simply reversing course after exiting the distal end of the tube and traveling back up and around the tube to exit the mouth. While treatment of this sort has proved successful for patients having chronic or acute respiratory diseases, there is a constant risk of several complications.

One of the most common complications in mechanical ventilation is known as ventilator associated (or acquired) pneumonia or VAP. Patients receiving tracheal intubation sometimes develop this pneumonia from an infection of the lungs, possibly induced by contaminated secretions, mucus or biofilm entering the trachea and the lungs after growing in the warm, moist environment in the central lumen of the tracheal tube. Removing these secretions from the tracheal tube lumen would likely reduce the risk of such infections.

In addition, it has been reported that extubated endotracheal tubes had significantly decreased luminal volume and radius compared to unused tubes. Even small changes in the luminal radius result in large changes in resistance to airflow-leading to an increased work of breathing, difficulty in breathing and increased length of hospital stays. The build-up of tenacious secretions within the tracheal tube can lead to difficulty in weaning off the mechanical ventilator, the need for emergency tracheal tube replacement, or the need for tracheostomy, all of which place the patient at greater risk of additional complications.

A number of attempts have been made to develop cleaning mechanisms for the central lumen of tracheal tubes. UK patent application GB 2482618 to Airway Medix Spolka Z.O.O. discusses a cleaning device having a balloon on the distal end and having a source of pressurized liquid and a source of suction to wash the interior of the central lumen and remove the liquid and biofilm. US patent 8,157,919 to Endoclear LLC provides a medical tube cleaning apparatus with a mechanically actuated, non-inflatable cleaning member. No liquid or suction are used.

Further known devices are disclosed in US 2011/023885 and WO 94/03226 which disclosed a device according to the preamble of claim 1.

What is needed is a mechanism for thorough cleaning of the central tracheal tube lumen.

### SUMMARY

In accordance with the invention, there is a device provided as set forth in claim 1.

This disclosure relates to a device (cleaning device, self-positioning cleaning device, or self-positioning tracheal tube cleaning device) for cleaning the interior walls of a breathing lumen, e.g., a catheter or a tracheal tube.

The whisks can change position with respect to the cleaning lumen, for example, each whisk has a portion that is capable of being in proximity to the cleaning lumen when compressed, but being distanced away from the cleaning lumen when unconstrained. The removal element self-positions the device concentrically within the tracheal tube. Suction is desirably applied to the cleaning lumen during use.

### BRIEF DESCRIPTION OF THE DRAWINGS

Figure 1 shows a cleaning device with whisks in a second position as it is withdrawn from a tracheal tube.
Figure 2 shows a cleaning device with whisks in another second position. The cleaning lumen, distal port, whisks and lateral openings are clearly visible. The whisks in this second position are in close proximity to the cleaning lumen.
Figure 3 shows a cleaning device having whisks in an unconstrained position, i.e. the first position, as is typical outside the tracheal tube. The cleaning lumen, distal port, whisks and lateral openings are clearly visible.

### DETAILED DESCRIPTION

Reference will now be made in detail to one or more embodiments, examples of which are illustrated in the drawings. It should be understood that features illustrated or described as part of one embodiment may be used with another embodiment to yield still a further embodiment.

Suction catheters are well known and widely commercially available for many medical uses. Suctioning may be performed using an "open" or "closed" system. In the open system, the suction catheter is merely a flexible plastic tube that is inserted into the tracheal tube breathing lumen with a source of suction connected to the proximal end of the suction catheter. Anything that the suction catheter touches before entering the lumen is preferably maintained in a sterile condition so a "sterile field" is created on or next to the patient. The suction catheter must be carefully handled after it is used since it will be coated with the patient's secretions. In contrast, in the "closed" system, for example that disclosed in US patent 4,569,344, a device which may be used to suction secretions is enclosed within a generally cylindrical plastic bag to eliminate or minimize contamination of the suction catheter prior to use. This is generally referred to as a "closed suction catheter" and is available under the trade name TRACH CARE ® (BALLARD® Medical Products) from Kimberly-Clark Corporation.

Disclosed is a device that enters the tracheal tube either by opening the ventilation circuit or by entering through an access port that gives access to the tracheal tube. The device has a proximal end, a distal end, and the removal element between these ends. The distal end of the device enters the tracheal tube first. The device may contain markings which indicate its advancement through the tracheal tube and may convey to the user information about the location of the device, e.g., when the distal end of the device reaches the distal end of the tracheal tube. The cleaning lumen of the device must of course be smaller than the interior diameter of the tracheal tube. The removal element must deform to fit within the interior of the tracheal tube. The whisks take on a second position when the device is inserted within the interior of the tracheal tube. The proximal collar reversibly slides along a portion of the exterior of the cleaning lumen when the whisks transition between the first and second positions.

The removal element comprises a plurality of whisks that are non-inflatable. The whisks strive to take on the first position, the unconstrained position, due to an intentional bias for a portion of each whisk to be a predetermined maximum distance away from the exterior of the cleaning lumen. When within the tracheal tube the whisks take on a second position such that portions of each whisk contact the interior of the tracheal tube.

Suction is desirably applied to the cleaning lumen during use.

The removal element self-positions the device to be generally concentric with the tracheal tube when the cleaning lumen is within the tracheal tube. This self-positioning is caused by the bias of the whisks, their spacing around the cleaning lumen, and the radial dimensions of the cleaning lumen and the tracheal tube interior.

The removal element has a maximum radial dimension in the first position. In second positions the removal element has its largest radial dimension less than the maximum of the first position. This change occurs in response to constraining forces applied to the whisks, e.g., within the tracheal tube; this change allows the cleaning device to fit through openings that are smaller than the maximum radial dimension of the removal element. The transition (change) between the first and second positions is repeatedly reversible.

The whisks are configured around the cleaning lumen and their distal ends are anchored to the cleaning lumen via the distal collar. The proximal ends of the whisks are maintained in close proximity to the cleaning lumen via their attachment to the sliding proximal collar. The proximal collar moves from a first location to a second location with respect to the distal collar when the whisks move from one constrained condition to another, i.e., different second positions, or to the unconstrained position, and vice versa. As a consequence of their attachment, the whisk proximal ends follow the location changes of the proximal collar and the intermediate whisk portions adjust their radial dimensions, while the distal whisk ends remain fixed in place.

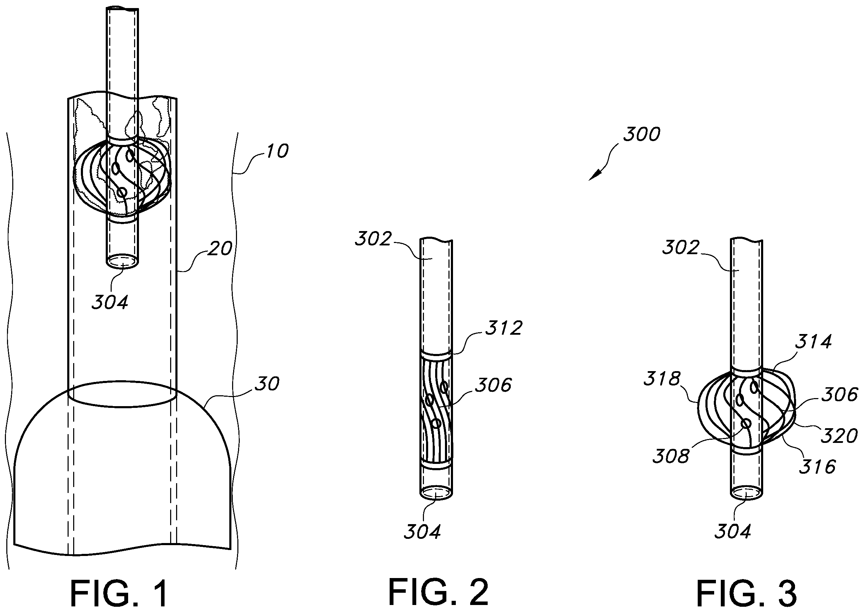

In the conventional use of an endotracheal tube, air is delivered to the patient's lungs through the breathing channel or lumen inside the tube 20. The tube 20 has a balloon cuff 30 that desirably seals against the trachea 10 such that secretions above the cuff and outside the tube do not move downwardly into the lungs (Figure 1). Further discussion of the functioning to the balloon cuff may be found, for example, in US patent 6,802,317 to Goebel. Mucus may nevertheless build up within the breathing channel or lumen of the tube, causing a decrease in the cross-sectional area of the lumen, thus increasing the resistance to air flow within the lumen and so decreasing the air flow to the patient's lungs. The mucus may also harbor unwanted bacteria that may thrive in the warm, moist environment inside the tube.

Figure 1 shows a self-positioning tracheal tube cleaning device 300 advancing within a tracheal tube, with the whisks 306 constrained in a second position against the cleaning lumen 302. This device 300 has a port 304 on the distal end of the cleaning lumen 302 and lateral ports 308 that are in fluid communication with the interior of the cleaning lumen. The device 300 has a removal element comprising whisks 306 spaced apart individually or in clusters around the cleaning lumen 302, the whisks 306 are fixed to the cleaning lumen 302 at a distal collar 310 but not fixed to the cleaning lumen 302 at a proximal collar 312. It is believed that a minimum of three individual whisks or three clusters of an equal number of whisks are needed to self-center the cleaning device and that they should be equally spaced about the cleaning lumen, e.g. 120 degrees from each other when there are only three whisks or whisk clusters. The cleaning device 300 may be a modified closed suction catheter as described above. The cleaning device 300 wipes the interior of the tracheal tube and removes secretion build-up every time it is retracted within the tracheal tube. The wiping is a result of whisk portions contacting the interior wall of the tracheal tube; the contact increases as the proximal collar slides towards the distal collar.

The whisks 306 around the cleaning lumen 302 are biased to an unconstrained state, the first position, in which the whisks 306 has a maximum radial dimension. Within the tracheal tube 20 the whisks span the distance between the interior wall of the tracheal tube and the exterior surface of the cleaning lumen 302. Each whisk 306 has a distal end 316 and a proximal end 314 with a bending region 318 and an intermediate portion 320 between these ends. The bending region 318 and the intermediate region 320 can be the same. The stiffness of the whisks 306 may vary between the distal and proximal ends 316, 314 to encourage the whisks to move towards the first position, but allow moving to and from various second positions. For example, the whisk portion near the distal end 316 (or distal collar) may be more stiff than the whisk portion near the proximal end 314 (or proximal collar) to allow the proximal collar 312 to more easily move downwardly as the cleaning device is withdrawn from the trach tube. Each of the whisks 306 can have a uniform or a varying cross-sectional shape between the proximal and distal collars, e.g., the cross-sectional shape can be bowed or curved with the concave portion facing towards the exterior of the cleaning lumen 302. The whisks may also taper in dimensions from their intermediate portions to one or both ends.

The distal ends 316 are fixedly attached to the exterior of the cleaning lumen 302 at the distal collar 310. The proximal ends 314 are connected to each other directly or by intermediate material to form a proximal collar 312 that encircles the cleaning lumen 302 but is not attached to the cleaning lumen 302. The proximal collar 312 reversibly slides along the cleaning lumen 302 between a maximum and minimum limit as the whisks 306 change from their biased constrained state. The maximum limit is where the proximal collar 312 is most proximally positioned as allowed by the collective whisks 306 and the respective whisk ends 314, 316 are farthest apart. Conversely, the minimal limit is where the proximal collar 312 is nearest the location where the distal ends 316 of the whisks 306 are attached to the cleaning lumen 302.

The whisks 306 on the sides of the cleaning lumen 302 can straighten with respect to the axial direction of the device 300 as device 300 is moved through the tracheal tube since the proximal collar 312 of the whisks 306 is not fixed on the cleaning lumen 302. As the distal movement of the device within the tracheal tube ceases, the bias of the whisks (to assume the first position) forces the whisks 306 to move outwardly from the cleaning lumen. As a result of the bias, the whisks 306 contact the inner walls of the tube as the device 300 and this contact increases as device 300 is withdrawn, loosening any deposits and directing them toward the lateral ports 308. The contact of the whisks 306 with the inner wall of the tracheal tube is enhanced when the bending region 318 of each whisk 306 attains the unconstrained state or enhances the bowing of the whisks 306 beyond the unconstrained state. Suction applied to the proximal end (not shown) of the device 300 pulls the deposits into the cleaning lumen 302 through the lateral ports 308 and the distal port 304.

Figures 2 and 3 show the cleaning device 300 with the cleaning lumen 302, distal port 304, whisks 306 and lateral openings 308 components clearly visible. The whisks 306 are in a maximally constrained second position in Figure 2 and in an unconstrained position, i.e., the first position, in Figure 3. The whisks 306 change positions when the proximal collar 312 moves from a first location to a second location with respect to the distal collar, i.e. it moves distally when intermediate portions of whisks 306 bend outwardly from the cleaning lumen 302. When the intermediate portions of the whisk contact the interior wall of the tracheal tube, the whisks 306 self-position the device 300 generally in the center of the tracheal tube breathing lumen. The cleaning device 300 may optionally have a film (not shown) covering the distal half of the whisks 306 to assist in catching any mucus that may escape the suction port 304 and lateral openings 308.

## Claims

1. A device (300) for cleaning the interior wall of a catheter (20) having deposits comprising a cleaning lumen (302), a removal element having whisks (306) capable of changing from a first, unconstrained positioned to a second, constrained position, each whisk (306 having a distal end (316) and a proximal end (314),
wherein the distal ends (316) of the whisks (306) are fixed to the cleaning lumen (302) at a distal collar (310), **characterised in that** the proximal ends (314) of the whisks (306) are connected to each other to form a proximal collar (312), and
wherein the proximal collar (312) encircles the cleaning lumen (302) but is not attached to the cleaning lumen (302).

2. The device (300) of claim 1 wherein said whisks (306) each have a portion that has a bias to deflect away from the cleaning lumen (302).

3. The device (300) of 1 wherein said cleaning lumen (302) has a distal port (304) and lateral ports (308), the whisks (306) are spaced apart individually or in clusters around the cleaning lumen (302), and wherein suction applied to a proximal end of said cleaning lumen (302) pulls said deposits through said lateral (308) and distal ports (304).

4. The device (300) of claim 1 wherein a whisk portion near the distal collar (310) is stiffer than a whisk portion near the proximal collar (312).

5. The device (300) of claim 3 having a film covering a distal half of the whisks (306) to assist in catching any deposits that may escape the distal (304) and lateral ports (308).

6. The device (300) of claim 2 wherein said whisks (306) are configured such that in the second position they center the device wihin the catheter (20) with respect to an interior wall of the catheter (20).

## Patentansprüche

1. Vorrichtung (300) zum Reinigen der Innenwand eines Ablagerungen aufweisenden Katheters (20), umfassend ein Reinigungslumen (302), ein Entfernungselement mit Wischern (306), die sich von einer ersten, uneingeschränkten Positionierten zu einer zweiten, eingeschränkten Position ändern können, wobei jeder Wischer (306 ein distales Ende (316) und ein proximales Ende (314) aufweist,
wobei die distalen Enden (316) der Wischer (306) an dem Reinigungslumen (302) an einem distalen Kragen (310) fixiert sind,
**dadurch gekennzeichnet, dass**
die proximalen Enden (314) der Wischer (306) miteinander verbunden sind, um einen proximalen Kragen (312) zu bilden, und
wobei der proximale Kragen (312) das Reinigungslumen (302) umgibt, aber nicht an dem Reinigungslumen (302) befestigt ist.

2. Vorrichtung (300) nach Anspruch 1, wobei die Wischer (306) jeweils einen Bereich aufweisen, der eine Vorspannung aufweist, die von dem Reinigungslumen (302) weg auslenkt.

3. Vorrichtung (300) nach 1, wobei das Reinigungslumen (302) einen distalen Port (304) und laterale Ports (308) aufweist, die Wischer (306) jeweils voneinander beabstandet sind, oder in Clustern um das Reinigungslumen (302) vorliegen, und wobei an ein proximales Ende des Reinigungslumens (302) Sog angelegt wird, um die Abscheidungen durch die lateralen (308) und distalen Ports (304) abzuziehen.

4. Vorrichtung (300) nach Anspruch 1, wobei ein Wischerbereich nahe dem distalen Kragen (310) steifer ist als ein Wischerbereich nahe dem proximalen Kragen (312).

5. Vorrichtung (300) nach Anspruch 3, mit einem Film, der eine distale Hälfte der Wischer (306) abdeckt, um beim Erfassen von beliebigen Abscheidungen, die den distalen (304) und lateralen Ports (308) entgehen, zu unterstützen.

6. Vorrichtung (300) nach Anspruch 2, wobei die Wischer (306) derart ausgelegt sind, dass sie in der zweiten Position die Vorrichtung in dem Katheter (20) hinsichtlich einer Innenwand des Katheters (20) zentrieren.

## Revendications

1. Dispositif (300) pour nettoyer la paroi intérieure d'un cathéter (20) ayant des dépôts, comprenant une lumière de nettoyage (302), un élément de retrait ayant des fouets (306) capables de passer d'une première position, non contrainte à une seconde position, contrainte, chaque fouet (306) ayant une extrémité distale (316) et une extrémité proximale (314),
dans lequel les extrémités distales (316) des fouets (306) sont fixées à la lumière de nettoyage (302) sur une bague distale (310),
**caractérisé en ce que** les extrémités proximales (314) des fouets (306) sont reliées l'une à l'autre pour former une bague proximale (312), et
dans lequel la bague proximale (312) encercle la lumière de nettoyage (302) mais n'est pas attaché à la lumière de nettoyage (302).

2. Dispositif (300) selon la revendication 1, dans lequel lesdits fouets (306) ont chacun une portion qui a un rappel pour dévier en s'éloignant de la lumière de nettoyage (302).

3. Dispositif (300) selon la revendication 1, dans lequel ladite lumière de nettoyage (302) a un orifice distal (304) et des orifices latéraux (308), les fouets (306) sont espacés individuellement ou en groupes autour de la lumière de nettoyage (302), et dans lequel une succion appliquée à une extrémité proximale de ladite lumière de nettoyage (302) tire lesdits dépôts à travers lesdits orifices latéraux (308) et distal (304).

4. Dispositif (300) selon la revendication 1, dans lequel une portion de fouet proche de la bague distale (310) est plus rigide qu'une portion de fouet proche de la bague proximale (312).

5. Dispositif (300) selon la revendication 3, ayant un film recouvrant une moitié distale des fouets (306) pour aider à capturer tout dépôt qui pourrait s'échapper des orifices distal (304) et latéraux (308).

6. Dispositif (300) selon la revendication 2, dans lequel lesdits fouets (306) sont configurés de telle sorte que dans la seconde position, ils centrent le dispositif à l'intérieur du cathéter (20) par rapport à une paroi intérieure du cathéter (20).
